# EUROPEAN PATENT SPECIFICATION

(11) **EP 0 618 813 B1**
(45) Date of publication and mention of the grant of the patent: **09.01.2002**
(21) Application number: 93922202.2
(22) Date of filing: 15.09.1993
(51) Int. Cl.: A61K 39/02, A61K 39/09, C07K 2/00, C07H 15/12, C07K 14/315, C07K 14/245

(54) **ANTIGEN OF HYBRID M PROTEIN AND CARRIER FOR GROUP A STREPTOCOCCAL VACCINE**
ANTIGENE DES HYBRIDEN M-PROTEINS UND TRÄGER FÜR GRUPPE A STREPTOKOKKENIMPFSTOFF
ANTIGENE DE LA PROTEINE M HYBRIDE ET PORTEUR DESTINE AU VACCIN ANTI-STREPTOCOCCIQUE DU GROUPE A

(30) Priority: 16.09.1992 US 945860
(43) Date of publication of application: 12.10.1994
(73) Proprietor: THE UNIVERSITY OF TENNESSEE RESEARCH CORPORATION, Knoxville, Tennessee 37966-0344 (US)
(72) Inventor: DALE, James, B., Memphis, TN 38111 (US)
(74) Representative: Gowshall, Jonathan Vallance
(86) International application number: PCT/US93/08704
(87) International publication number: WO 94/06465

(56) References cited:
- WO-A-85/00832
- WO-A-93/21220
- US-A- 4 284 537
- US-A- 5 124 153
- J IMMUNOL 151 (4). 1993. 2188-2194. CODEN: JOIMA3 ISSN: 0022-1767, XP000645174 DALE J B ET AL: "RECOMBINANT TETRAVALENT GROUP A STREPTOCOCCAL M PROTEIN VACCINE."
- GINSBERG, H. S., ET AL. (ED.). VACCINES (COLD SPRING HARBOR), 93. MODERN APPROACHES TO NEW VACCINES INCLUDING PREVENTION OF AIDS;TENTH ANNUAL MEETING, COLD SPRING HARBOR, NEW YORK, USA, SEPTEMBER 1992. XXV+454P. COLD SPRING HARBOR LABORATORY PRESS: P, XP000618952 DALE J B ET AL: "PROTECTIVE IMMUNOGENICITY OF A RECOMBINANT HYBRID PROTEIN CONTAINING A FRAGMENT OF TYPE-24 STREPTOCOCCAL M PROTEIN AND THE B SUBUNIT OF ESCHERICHIA-COLI LABILE TOXIN."
- SCIENCE, vol. 244, no. 4911, 23 June 1989, WASHINGTON, D.C. , pages 1487-1490, XP000645183 V.A. FISCHETTI ET AL.: "Protection against Streptococcal pharyngeal colonization with a Vaccinia: M protein recombinant."
- VACCINE, vol. 6, no. 2, April 1988, pages 192-196, XP000645184 E. H. BEACHEY ET AL.: "Protective and autoimmune epitopes of streptococcal M proteins"
- J. EXP. MED. , vol. 163, no. 6, June 1986, pages 1451-1458, XP000645526 E. H. BEACHEY ET AL.: "Opsonic antibodies evoked by hybrid peptide copies of types 5 and 24 Streptococcal M proteins synthesized in tandem."
- INFECT IMMUN 56 (8). 1988. 2198-2204. CODEN: INFIBR ISSN: 0019-9567, XP000645176 MILLER L ET AL: "CONSERVATION OF PROTECTIVE AND NONPROTECTIVE EPITOPES IN M PROTEINS OF GROUP A STREPTOCOCCI."
- J. Exp. Med. 166, issued September 1987, E.H. BEACHEY et al., "Protective immunogenicity and T lymphotocyte specificity of a trivalent hybrid peptide containing NH2-terminal sequences of types 5, 6, and 24 in Proteins synthesized in tandem", pages 647-656, entire document.
- BRAUDE et al., "Infectious Diseases and Medical Microbiology", published 1986 by W.B. Saunders Company (PA), pages 242-253, entire document.

## Description

### FIELD OF INVENTION

The present invention relates broadly to the field of recombinant vaccines. The vaccines are directed to preventing Group A streptococcal infections, which may otherwise result in rheumatic fever.

### BACKGROUND OF THE INVENTION

Acute rheumatic fever (ARF) is the major cause of heart disease in children around the world. The disease is rampant in developing countries where prevalence rates of rheumatic heart disease may be as high as 35-40 per thousand individuals. By one estimate, it affects nearly six millon school-age children in India. Although the incidence of ARF in the United States and other Western countries declined markedly during the later half of the twentieth century, there has been a remarkable resurgence of the disease in the United States.

Streptococci are a group of bacteria with the capacity to grow in chains. Many varieties are part of the normal bacterial flora in humans and are not especially harmful. However, a particular subgroup of streptococcal bacteria, called Group A and represented by Streptococcus pyogenes, is a human pathogen. Between 20 and 30 millon cases of Group A streptococcal infections occur every year in the United States alone. These cases include infections of the skin and throat, forms of pneumonia and a recently identified disease resembling toxic shock. The most common infection is acute streptococcal pharyngitis, or strep throat, which occurs predominantly in school-age children. Strep throat qualifies as a major worldwide health problem if judged only by time lost from school and work and by the amount spent on related doctor's fees.

Strep throat's toll is much greater, however. In as many as 4% of the pharyngitis cases that are untreated or treated ineffectively, the strep infection leads to ARF. Current attempts to prevent ARF rely on treatment of the pharyngitis with antibiotics. During a recent outbreak of ARF in Utah, only a fourth of the patients sought health care prior to the onset of symptoms, and only a third recalled a recent sore throat. The finding that ARF may follow a subclinical infection in such a high percentage of individuals and the fact that access to health care in developing countries is not widely available serve to underscore the need for a safe and effective vaccine against Group A streptococci.

The causal relationship between streptococcal pharyngitis and ARF was established over 50 years ago, yet the mechanism of the pathogenesis of the disease remains unclear. It is widely held that ARF is an autoimmune disease, and that in the susceptible host the infection triggers an immune response that leads to inflammatory and sometimes destructive changes in target tissues. Streptococci have been shown to contain antigens that are immunologically cross-reactive with host tissues (14, 20, 24, 26, 27, 35, 39 40, 41, 44, 55) and heart-crossreactive antibodies from patients with rheumatic fever have been shown to react with streptococci (15). However, it was also shown that sera from patients with uncomplicated pharyngitis also may contain heart-crossreactive antibodies (57), yet these patients do not develop clinical evidence of carditis. Until the significance of tissuecrossreactive antibodies in the pathogenesis of ARF is better understood, there remains a need to exclude potentially harmful epitopes from vaccine preparations.

The surface M protein of Group A streptococci is the major virulence factor and protective antigen of these organisms (45). Group A streptococci have developed a system for avoiding some of the antimicrobial defenses of a human host. Strains of streptococci that are rich in M protein evade phagocytosis by PMNs and multiply in non-immune blood (45). Yet, resistance to an infection by these bacteria is possible if the host's body can produce opsonic antibodies directed against the M protein. Such antibodies will neutralize the protective capacity of the M protein and allow the streptococcus to be engulfed and destroyed by phagocytes. The development of secretory or mucosal immunity is also now suspected of playing an important role in preventing streptococcal infections.

A major obstacle to effective vaccine development has been the tremendous number of M protein serotypes (now over 80) (33). Laboratory tests suggest that antibodies against one serotype do not offer protection against others. Immunity then appears to be type or sero-specific and optimal vaccines would require that most of the serotypes be represented. There is evidence that not all serotypes of Group A streptococci have the same potential to trigger acute rheumatic fever in susceptible individuals (12). The concept of "rheumatogenic" and "son-rheumatogenic" organisms is supported by multiple surveillance studies over many years and in diverse areas of the world. Thus, there are probably about 12-15 serotypes responsible for most cases of ARF. Some of these are types 1, 3, 5, 6, 14, 18, 19, 24, 27 and 29.

Previous studies have shown that in many cases the protective epitopes of M protein may be separated from the potentially harmful, autoimmune epitopes of the molecule (4, 5, 7, 23, 29). The NH₂-terminal segments of M proteins have evoked antibodies with the greatest bactericidal activity (5, 23, 38).

Previous studies have also shown that synthetic peptides copying limited regions of types 5, 6 and 24 M proteins evoked type-specific, opsonic antibodies that were not heart tissue cross-reactive (4, 5, 23). Because of their lack of immunogenicity (haptens), the synthetic peptides were chemically linked covalently to carrier proteins (4, 5, and 23). However, such fragments of M proteins linked to carrier proteins with chemical reagents do not result in hybrid proteins of defined structures. Thus, in general it has not been possible to obtain antigens which can elicit specific, desired antibodies or which decrease the risk of undesirable side reactions. Further, formation of hapten - carrier complexes using chemical cross-linking reagents is time-consuming and costly and results in undefined heterogenous mixtures of vaccine components.

It is evident from this description of the state of the art that there is an important need for a vaccine which is effective by raising sero-specific antibodies against the various serotypes of Group A streptococci, especially those serotypes capable of triggering acute rheumatic fever, which is known to follow a sore throat, without eliciting cross-reaction with human tissue. Particularly, there is an important need for a vaccine which has not only these properties, but which also is capable of raising protective antibodies to prevent sore throat, skin infections, deep tissue infections and streptococcal infections of the like that are not necessarily followed by rheumatic fever. The invention contributes to solving these important needs in human health.

### SUMMARY OF THE INVENTION

This patent application is related to and is co-filed on the same day as patent application No. EP-A-0625043 entitled "RECOMBINANT MULTIVALENT M PROTEIN VACCINE" with named inventors James B. Dale and James W. Lederer.

The present invention provides recombinant M protein antigens. The antigens are constructed by recombinant DNA methods. They are comprised of amino acid fragments of serotypes of M protein at least one of which is serotype M1, which fragments carry one or more epitopes that evoke opsonic antibodies against specific serotypes of Group A streptococcus and, if desired, when the fragments carry appropriate epitopes, also evoke protective antibodies. The fragments are either fused directly or linked in tandem by an amino acid linker to an appropriate carrier. The antigens are generally non-immunogenic (or not adequately immunogenic) because of their molecular size or for other reasons.

The invention thus provides a recombinant fusion antigen comprising a gene encoding the carrier protein and an NH₂ or COOH-terminal M protein fragment carrying one or more epitopes. The recombinant antigen does not elicit antibodies which cross react with human heart or other human tissue.

In accordance with the invention, there are provided mixtures of antigens which are serotype-specific comprising the same or different carrier. Such a mixture of selected antigens-carriers or "cocktail" provides immunogenicity against several serotypes (and if desired raise different protective antibodies). The recombinant fusion antigens are constituted of segments of the NH₂ terminal portions of the M proteins, which fragments raise specific opsonic antibodies. Fusion antigens are also provided which are constituted of the COOH-terminal fragments of the M proteins. The COOH-terminal fragments raise protective antibodies of the mucosal or secretory type. In the antigen with an amino acid linker, the carrier and the fragment of the M protein, which carries the desired epitope, are linked in tandem by an amino acid linker, described in greater detail hereinafter, which has the capacity to promote the conformation of the fragment of the M protein to optimize the exposure of the epilope and thus to optimally raise the desired antibodies.

The invention also provides for an antigen comprised of a carrier, which constitutes the carboxy-terminal portion of a serotype of M protein linked by a linker or fused directly to an amino acid fragment of M protein. The carrier and fragment may be of the same or different serotype wherein at least one serotype is M1.

There are also disclosed carriers which are free of epitopes which elicit antibodies to serotypes of streptococcal M protein.

There are disclosed recombinant hybrid genes which nucleotide sequences encode for the antigens of the present invention and a method of construction of such genes.

The invention further provides the new fusion genes or DNA fragments which code for the hybrid antigens and the transformed microorganisms (eukaryotes or prokaryotes) that express the hybrid antigens.

There are disclosed avirulent microorganisms which are transformed with the genes of the present invention. These microorganisms are especially suitable for oral administration to and immunization of mammals, in particular humans.

There are disclosed methods of administration of the antigens of the present invention in therapeutic compositions via oral, intranasal and parenteral routes of administration, to induce or evoke opsonic and/or protective antibodies against serotypes of Group A streptococcus. The administered compositions confer immunity to immunized mammals against Group A streptococci.

The invention provides vaccine compositions which are complised of the antigens of the present invention and biologically acceptable diluents for administration to and immunization of mammals, in particular humans. The composition is administrable orally, whereby the antigens are released from the transformed microorganism and the desired antibodies are elicited, intranasally and parenterally.

There is disclosed broad spectrum protection and wide-ranging immunity against all serotypes of Group A streptococci, particularly rheumatogenic streptococci by the formulation of compositions of the antigens, either singly or in mixtures or "cocktails".

### BRIEF DESCRIPTION OF THE FIGURES

Figure 1 shows the DNA and deduced protein sequence of LT-B-M24 hybrid molecule.

Figure 2 shows the immunoblot analysis of purified LT-B-M24 hybrid protein.

Figure 3 shows the immunogenicity of LT-B-M24 in rabbits, as determined by ELISA.

Figure 4 shows the order of the nucleotides and amino acid residues of an antigen of a fragment of M5 and a carrier of the COOH-terminal portion of M5.

Figure 5 shows the order of the nucleotides and amino acid residues of an antigen of fragments of M5 and a carrier of the COOH-terminal portion of M5.

### DETAILED DESCRIPTION OF THE FIGURES

The present invention and many of the attendant advantages of the invention will be befter understood upon a reading of the following detailed description when considered in connection with the accompanying drawings wherein:

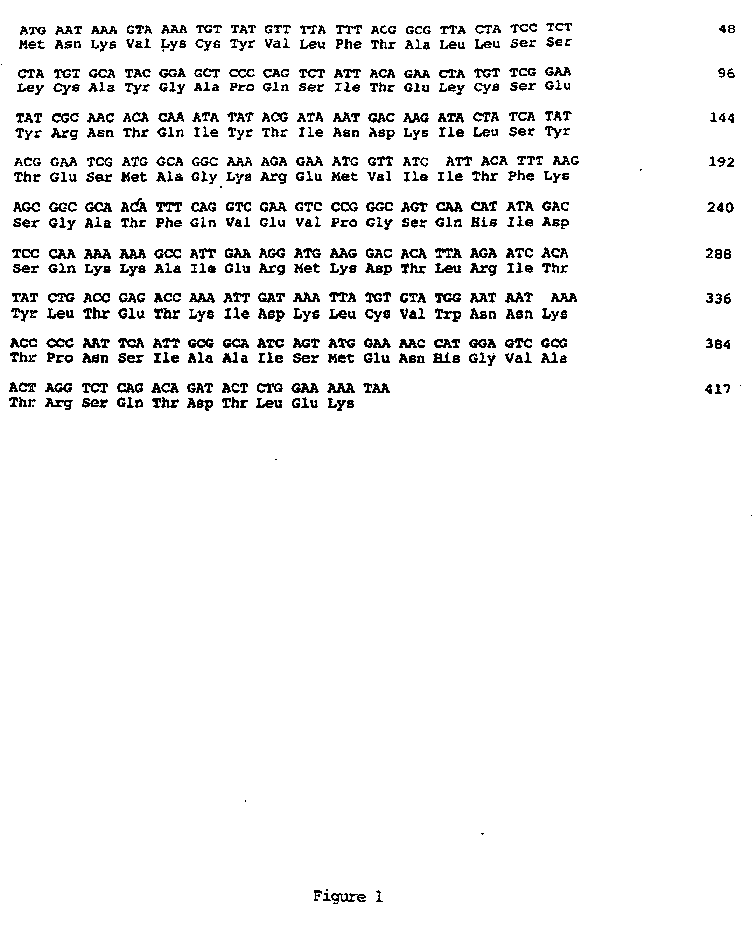

Figure 1 shows the DNA and deduced protein sequence of LT-B-M24 hybrid molecule. The sequence of the fusion gene was confirmed from base 228 to the 3' end. The remainder of the LT-B sequence is from Clements (16). The NcoI site linking the LT-B and M24 components is indicated. The M24 subunit is underlined.

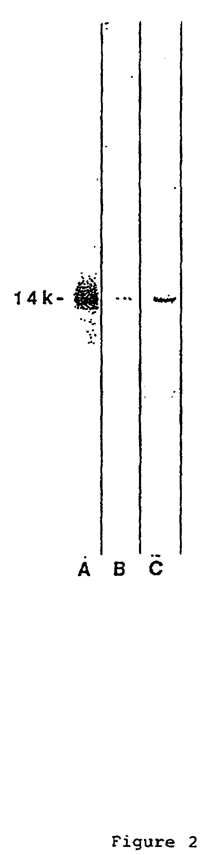

Figure 2 shows the immunoblot analysis of purified LT-B-M24 hybrid protein. The purified protein was electrophoresed on an SDS-polyacrylamide gel and transferred to nitrocellulose paper. Coomassie blue stained a single band with an apparent molecular weight of 14kDa (lane A). The purified protein reacted with rabbit antisera against LT-B (lane B) and SM24 (1-29)C (lane C).

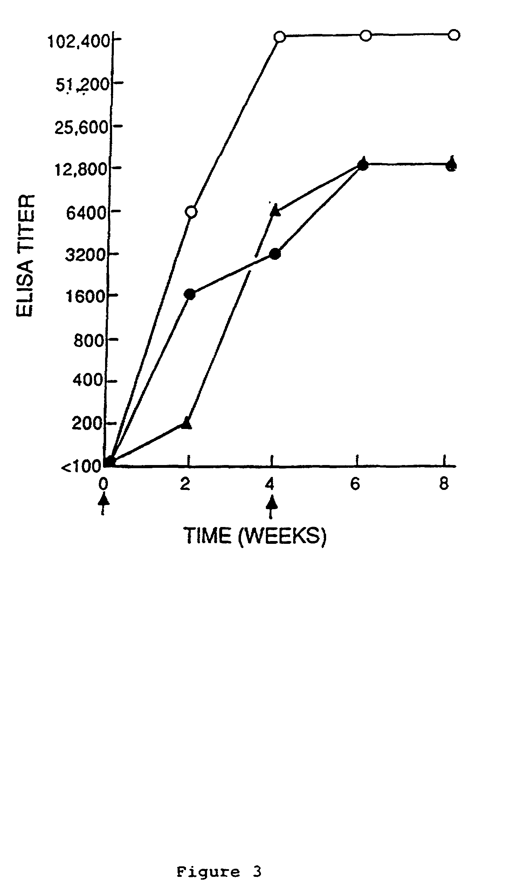

Figure 3 shows immunogenicity of LT-B-M24 in rabbits, as determined by ELISA. Three rabbits (o, **•**, Δ) were immunized with 300 µg LT-B-M24 at time 0 and at 4 weeks (arrows) and sera collected at two- week intervals were assayed for the presence of antibodies against pep M24 by ELISA. Titers are expressed as the reciprocal of last dilution of antiserum that resulted in an O.D. of >0.1 at 580 nm. ELISA performed at various intervals after the initial injection of LT-B-M24 revealed a brisk antibody response in all three rabbits, even after a single intracutaneous dose of LT-B-M24.

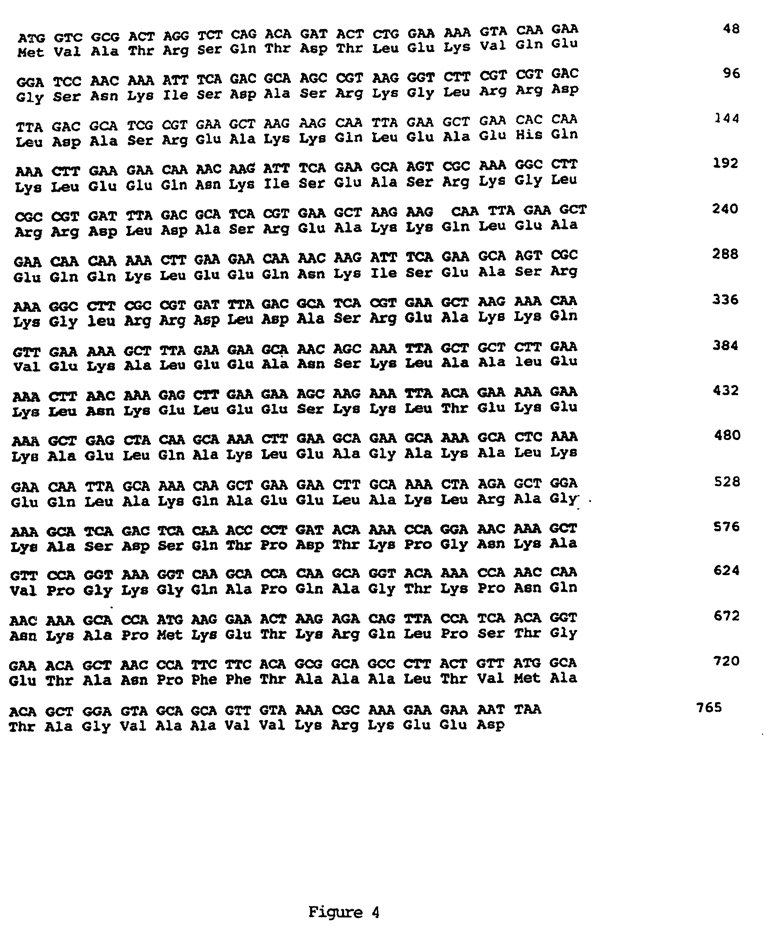

Figure 4 shows a construct of 762 nucleotides coding for an antigen of 254 amino acid residues, as shown. The antigen is comprised of an MS hapten fragment of 16 amino acids. The fragment is joined by a Bam H1 restriction site to a carrier, which is the COOH-terminal half of M5. The carrier includes 2.5 C-repeats, which each commence with the tetrapeptide Asn-Lys-Ile-Ser. While there is no amino acid linker linking the fragment and carrier, the Bam H1 is a suitable site for insertion of an appropriate linker.

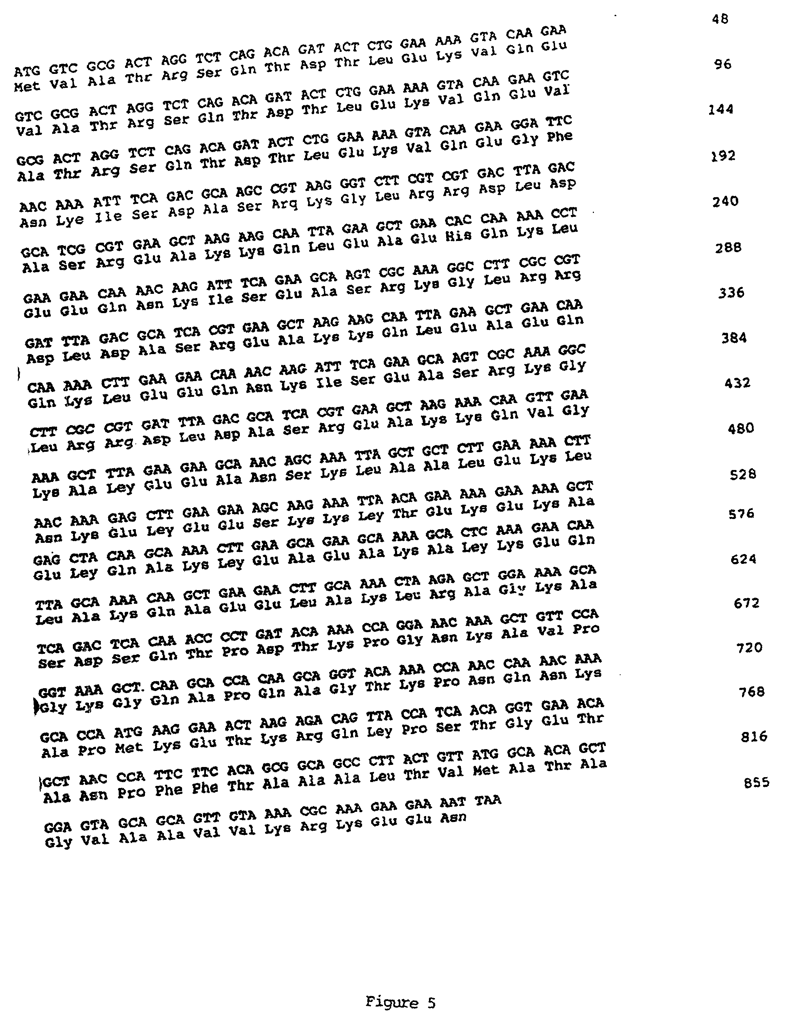

Figure 5 shows a construct of 852 nucleotides coding for an antigen of 284 amino acid residues, as shown. The antigen is comprised of three segments of M5 designated A, B and C, respectively. The C segment is joined by a Bam H1 restriction site to a carrier, which is the COOH-terminal half of M5. The carrier includes 2.5 C-repeats, which each commence with the tetrapeptide Asn-Lys-Ile-Ser. While there is no amino acid linker linking the fragment and carrier, the Bam H1 is a suitable site for insertion of an appropriate linker.

### DETAILED DESCRIPTION OF THE INVENTION

The above and various other aspects and advantages of the present invention are described hereinafter. All references above and hereinafter arc incorporated by reference, and identified in the Bibliography as numbered in the text.

The invention provides for a recombinant hybrid streptococcal M protein antigen which elicits opsonic antibodies to a serotype of Group A streptococcus wherein at least one serotype is M1, which comprises a carrier linked by an amino acid linker to an amino acid fragment, the fragment having at least one epitope of a serotype of Group A streptococcal M protein. The fragment elicits opsonic antibodies to the epitope or epitopes of the target serotype, without eliciting cross-reactive antibodies to mammalian heart tissue antigens.

It is also an embodiment of the present invention that the antigen of carrier and fragment are joined or fused together without the presence of a linker.

The amino acid fragment is of a portion of streptococcal M1 protein serotype of Group A streptococcus. The fragment is of a peptide size which is non-immunogenic (or inadequately immunogenic) by itself. In that sense, the fragment may be considered a hapten if it were considered by itself. Where a hapten is defined as a low molecular weight determinant group, which by itself is non-immunogenic, but which becomes so when placed on a larger "carrier" molecule (11). If coupled to a carrier protein of appropriate size, haptens are capable of inducing a strong immune response in an animal or human.

By the present invention, the recombinant hybrid proteins are selected to be constituted of amino-terminal (NH₂) fragments of M proteins which contain immunoprotective epitopes, which elicit opsonic antibodies to a target serotype of Group A streptococcal M protein at least one of which is M1. These fragments of the present invention do not contain cross-reactive or autoimmune epitopes to generate an autoimmune response directed to heart or other tissue antigens. As a consequence, the risk of developing ARF is minimized. The fragments of the M protein elicit antibodies of the opsonic or serum type which assist the phagocytic engulfment of the Group A streptococcus. The fragments of the M proteins may also elicit, if so desired, protective antibodies, which are secretory or mucosal antibodies, (e.g. from the saliva or nasal membranes), which play a critical role in a first-line defense against an invading streptococcus at the locus or site of infection. In that instance, the fragments are portions of the COOH-terminal half of M proteins, which fragments are free of epitopes which elicit antibodies which cross-react with heart or other tissues.

Thus two types of antibodies are elicited from epitopes located on two different portions of the M protein molecule. The NH₂-terminal fragments contain epitopes which elicit serum or opsonic antibodies in immunized mammals. The COOH-terminal fragments of the M protein elicit protective secretory or mucosal immunity in immunized mammals, e.g. IgA antibodies.

It should be noted in conjunction with the invention, as has been described herein, that not all M protein epitopes are sero-specific in their NH₂-terminal portion. Some epitopes of particular serotypes, such as M5, do cross-react to some extent with streptococci of a type other than M5, such as M6 or M19. And to some extent, this also occurs with other M serotypes. Accordingly, it is within the scope of the invention that when type-specificity is referred to, this does not exclude some cross-reactivity between certain shared structures of different serotypes. However, one should be careful to note that such shared epitopes that would cross-react with heart tissue are to be excluded from the selected fragments. Also included are NH-terminal fragments which, in addition to evoking opsonic antibodies, also contain epitopes that evoke protective secretory or mucosal antibodies.

A mixture or cocktail of antigens is provided. The antigens in the mixture contain NH₂ or COOH-terminal fragments of M protein of different serotypes which can elicit opsonic and protective antibodies, respectively, to a wide range of serotypes, particularly the rheumatogenic streptococci wherein at least one serotype is M1.

Thus, if it is desired in accordance with the invention to provide immunity against streptococcus infections that are likely to cause rheumatic fever, DNA molecules will be constructed which code for NH₂-or COOH-terminal fragments which contain epitopes that elicit opsonic or protective to antibodies types 1, 3, 5, 6, 14, 18, 19, 24, 27 or 29 M proteins, for instance and linked in tandem by an amino acid linker or fused directly with an appropriate carrier.

Examples of suitable NH₂-terminal fragments of M protein for constructing antigens, which elicit opsonic antibodies in an immunized animal are described hereinafter. For M24, there is provided a 15 amino acid fragment of the order Val-Ala-Thr-Arg-Ser-Gln-Thr-Asp-Thr-Leu-Glu-Lys-Val-Gln-Glu. For M5, there is,provided a 15 amino acid fragment of the order Ala, Val. Thr-Arg-Gly-Thr-Ile-Asn-Asp-Pro-Gln-Arg-Ala-Lys-Glu. For M6, there is provided a 15 amino acid fragment of the order Arg-Val-Phe-Pro-Arg-Gly-Thr-Val-Glu-Asn-Pro-Asp-Lys-Ala-Arg. For M19, there is provided a 15 amino acid fragment of the order Arg-Val-Arg-Tyr-Thr-Arg-His-Thr-Pro-Glu-Asp-Lys-Leu-Lys-Lys. For M3, there is provided a 15 amino acid fragment of the order Asp-Ala-Arg-Ser-Val-Asn-Gly-Glu-Phe-Pro-Arg-His-Val-Lys-Leu. For M1, there is provided a 15 amino acid fragment of the order Asn-Gly-Asp-Gly-Asn-Pro-Arg-Glu-Val-Ile-Glu-Asp-Leu-Ala-Ala. For M18, there is provided a 15 amino acid fragment in the order AIa-Pro-Leu-Thr-Arg-Ala-Thr-Ala-Asp-Asn-Lys-Asp-Glu-Leu-Ile. For M12, there is provided a 15 amino acid fragmentof the order His-Ser-Asp-Leu-Val-Ala-Glu-Lys-Glu-Arg-Leu-Glu-Asp-Len-Gly. These NH₂-terminal fragments will elicit opsonic antibodies in immunized animals when linked or fused to an appropriate carrier, which carrier may also favorably elicit desirable antibodies. These, as well as other antigens of the present invention may be administered to a mammal as a cocktail or mixture to elicit broad-spectrum antibodies. In such fashion, broad spectrum immunity is provided to immunized mammals against Group A streptococci.

ft is important to note that the invention is not limited to a particular amino acid sequence, wherever amino acid sequences are referred to or described, as above. In any particular amino acid sequence or fragment referred to herein, any one or more amino acids can be removed, substituted or replaced by some other amino acid(s), providing that the desired epitopes are not adversely affected by such changes in the primary structure of the protein fragments. Indeed, this is a quite common occurrence for the M protein among various strains within the same serotype. This tendency of variation in the sequence of the M protein has been shown for several such M proteins. (9 and 23).

Accordingly, any single fragment of a given serotype encoded by a gene may have its sequence altered, so as to carry multiple epitopes for a multitude of strains within the same serotype. In this fashion, a single antigen will elicit desirable antibodies to a number of strains within the same serotype. It is therefore an important concept of the invention that once reference is made to a particular serotype (i.e., of any one of the known or to be discovered serotypes, e.g., 1-82), reference is not intended to one single strain of a type, but to the various strains within the serotype. Thus, not only is it a fundamental concept of the invention to provide a vaccine of a cocktail or mixture against different serotypes, but also a vaccine directed to different strains within that particular serotype.

Thus, in accordance with the invention, there is provided a vaccine which is effective against not only different serotypes of M proteins, but also against various strains within the individual serotypes.

The amino acid linker sequences ideally contribute to the maximum accessibility of the epitopes of the fragments so as to generate the desired antibodies. Thus the linkers may function to influence the orientation, conformation or other aspects of the amino acid fragment -carrier molecule. Ideally, the linkers contribute to the orientation so that the hybrid molecule, or at least the amino acid fragment, mimics the native molecule.

The linker amino acid sequence is provided by construction of a gene coding for the desired M protein amino acid sequence and carrier with a restriction site to permit insertion of the DNA coding for the linker between the sequences of the fragment and the carrier, which linker links the carrier and fragment in tandem. Illustratively, such a linker is a proline-rich linker of Pro-Gly-Asn-Pro-Ala-Val-Pro. Other amino acid linkers may be used such as Ile-Pro-Gly, Asp-Pro-Arg-Val-Pro-Ser-Ser or His-Gly. An amino acid linker of Asp-Pro-Arg-Val-Pro-Ser-Ser or its equivalent is also considered suitable. While in theory, a linker could be constituted by one amino acid, so long as the desired immunoreactive conformation is achieved, longer linker regions may be more suitable to optimize the immunogenicity of the epitopes.

The effect of the different linkers on the immunogenicity of the hybrid molecule may justify further investigations. It is not excluded that, depending on the type and number of the amino acids of the linker, a hybrid antigen of ideal or close to ideal high immunogenicity may be identified.

A linker of hydrophohic acids is most desirable, such as tryptophan, alanine, Ieucine, isoleucine, valine, tyrosine, phenylalanine, proline, methionine and combinations thereof. The linkers may range in number from 2 to 30, providing that there is no adverse effect on the immunogenicity of the molecule. Quite satisfactory results have been obtained with a two amino acid linker of His-Gly, suggesting that such a small molecule may be satisfactory for M protein components. The sequence of the amino acids in any particular linker does not appear at this time to be critical.

As described further below, linkers are useful but not essential to join the carrier and fragment. The carrier and fragment may be joined or fused together directly.

Gene constructs are provided which encode an amino-terminal fragment of an M protein serotype linked with the COOH-terminal half of an M protein, which functions as a carrier. See Figures 4 and 5. As shown therein, a Bam H1 restriction site serves as a junction between the fragment and carrier. It is contemplated that the restriction site be a suitable site for insertion of an appropriate amino acid linker. The COOH-terminal carrier may elicit protective mucosal or secretory antibodies to serotypes of M protein. These antibodies may play an important role in preventing colonization and infection by Group A streptococci. Such a carrier thus serves a dual purpose, It serves the function of introducing the epitope(s) present on the amino-terminal hapten fragment to the macrophages for processing and antigen presentation to helper T cells for generating a type-specific humoral response. And the carrier possesses epitopes which may elicit more broadly protective IgA antibodies at the mucosal or secretory level. These antibodies are more broadly protective than the type-specific opsonic type, as they share more homologous epitopes among the various types of rheumatogenic streptococci. In this fashion, such protective antibodies are advantageously of a cross-protective or cross-serotype nature.

Instead of using the entire carboxyl-terminal of any one of the serotypes, it may be advantageous and desirable to only use one or more C-repeats of the M protein as a carrier. It is noteworthy that the carboxyl-terminal portion of M protein used in the construct need not be one of the same serotype as that which constitutes the amino terminal portion of the construct.

Instead of using the COOH-terminal portion or half of an M protein as a carrier, other suitable carriers for the fragments include surface proteins from gram-positive cocci (mostly from streptococci and staphylococci) (32). These proteins, which have been sequenced, are IgA binding protein (ARP 4), F_{c} binding protein (F_{c}RA), human IgG F_{c} binding protein (Protein H), C5a peptidase (SCP) and T6 surface protein from S. pyogenes; wall-associated protein (wap A) and cell surface protein (PAc and spa P) from S. mutans; Protein G, an IgG binding protein from Group G streptococci, Protein A and fibronectin binding protein (FnBP) from S. aureus, and a cell wall protease (wg 2) from S. cremoris. Beginning at the C-terminal end of these molecules, these proteins and the M proteins all have a similar arrangement of amino acids. Up to seven charged amino acids are found at the C-terminus which are composed of a mixture of both negative and positive charged residues. Immediately, N-terminal to this short charged region is a segment of 15-22 predominately hydrophobic amino acids. Beginning about nine amino acids N-terminal from the hydrophobic domain is found a hexapeptide with the consensus sequence LPSTGE, that is extremely conserved among all the proteins. Analysis of 12 of these surface molecules revealed that these proteins contain repeat segments, as in the M proteins, and were predominately helical within the region containing the repeat segments.

These proteins, as carriers, can be linked in tandem by an amino acid linker to NH₂ or COOH-terminal fragments or fused directly with the fragments, so long as the immunogenicity of the antigens is not adversely affected.

The amino acid fragments of the present invention are linked, either with or without a linker, to an appropriate carrier. For this purpose, any type of carrier is contemplated so long as the amino acid fragment linked to the carrier generates an opsonic or protective immune response to the epitopes of the fragment. The carrier may be a molecule which is free of an epitope which elicits antibodies to a serotype of streptococcal M protein. An example would be the B subunit E. coli labile toxin (LT-B). Alternatively, the carrier may be selected from the COOH-terminal portion of the M protein which is not cross-reactive with human tissue, and which may favorably elicit protective mucosal antibodies. Or the carrier may be the COOH-terminal portion of surface proteins of gram-positive cocci, as described above.

Other examples of suitable carriers may be keyhole limpet hemocyanin (KLH), tetanus toxoid, diphtheria toxoid, bovine serum albumin, hen egg lysozyme, gelatin, bovine gammaglobulin and flagellin polymer.

As shown in Figures 4 and 5, it is disclosed that the presence of linkers in the gene constructs, linking in tandem the fragment and carrier, is not required to elicit opsonic or protective antibodies in an immunized host. The fragment and carrier, in this embodiment of the invention, are fused directly to each other. A fusion antigen is suitable so long as the immunogenicity of the antigen is not adversely affected. The resulting gene constructs are fusion constructs.

Alternatively, a linker of amino acids may be added by chemical means after the antigen is expressed, e.g. by treatment with succinimidyl-4-(N-maleiamido-methyl) cyclohexone-1-carboxylate.

It is also contemplated that the constructs of the invention be constructed to contain a fragment of the NH₂ or COOH-terminal region of serotypes, which are not known to have a rheumatogenic effect, as those types described above and in the literature. In those instances, where such fragments have not yet been sequenced or not yet been published, whether rheumatogenic or non-rheumatogenic, one skilled in the art can sequence such structures by methods readily available. The invention also includes the construction of hybrid constructs containing repeating amino-terminal or carboxyl-terminal M protein fragments using PCR. One skilled in the art can utilize homologous regions of published M protein (emm) gene sequences from Group A streptococci (GAS; Streptococcus pyogenes) to design three primer pairs for PCR and three oligonucleotide probe sequences internal to the amplified products. One set of primers and corresponding probe can detect and lead to amplification of emm (-like) genes of virtually every type ("all M"). Another set ("SOR⁻M") amplifies only emm (-like) genes from GAS negative for serum opacity reaction (SOR). And a third set ("SOR⁻ M") expands only emm (-like) genes from SOR⁻GAS. Using the "all M" primer pair for PCR on the genomic DNA from gas of 29 different M types, as well as from a Group C and a Group G streptococcal isolate, DNA fragments within the expected size range were amplified in every assay. All PCR products reacted with the "all M" probe (49).

Thus, there is disclosed a hybrid fragment-carrier protein antigen encoded by an appropriate gene or genes to express in an appropriate organism, the antigen that will elicit the desired antibodies. Thus encompassed within the scope of the present invention are antigens comprised of opsonic antibody-generating NH₂ or COOH-terminal fragments of M protein from all the known rheumatogenic types of streptococci, and fragments from types of streptococci which are not, or at least not yet known or shown to be, associated with ARF. An example of a non-rheumatogenic streptococcal type, the M protein antigen of which is within the scope of the invention, is type 12.

The appropriate genes are constructed and expressed, as described hereinafter. The genes encoding the appropriate carriers of the present invention are inserted into appropriate plasmids. Non-limiting examples of the appropriate carriers are the carboxyl-terminal half of M proteins, the carboxyl-terminal half of surface proteins of gram-positive cocci and the B subunits of E. coli labile (LT-B) and cholera toxin (CT-B) from Vibrio cholerae. The plasmids are modified to contain a small polylinker with three endonuclease restriction sites at the 3' end, followed by transcription terminators in each reading frame (16). The selected genes encoding the desired NH₂ or COOH-terminal fragments of M protein are constructed in a suitable manner. For instance, a pair of oligonucleotides coding for the fragments are synthesized using an automated DNA synthesizer (ABI, model 381A). The desired oligonucleotides copy the appropriate first number of base pairs of the genes encoding the desired NH₂ or COOH-terminal fragments of M protein. Additionally, the oligonucleotides encompass the right hand side of an appropriate restriction site at the 5' end, for instance NcoI. The oligonucleotides are mixed in equimolar ratios, heated to an appropriate temperature and allowed to anneal at ambient temperature. The appropriate plasmids are digested with restriction enzymes, and the cut plasmids are then purified. For instance, plasmid pPx1604 was digested with NcoI and Eco RV and purified from agarose gels over glassmilk (Geneclean, Bio 101, La Jolla, Ca). The synthetic oligonucleotide pairs of interest are then ligated into the cut sites of the plasmids. The plasmids containing the M protein fragments of interest are then used to transform an appropriate microorganism. For instance, E. coli JM105 is a suitable microorganism. Transformants are then screened by an appropriate method, e.g. dot blot analysis using appropriate antisera.

For high level expression of the M protein antigens of the present invention, insertion of the selected gene constructs, encoding the antigens, into suitable plasmids is carried out. An example of a suitable plasmid is pKK223-3 (Pharmacia, Uppsala, Sweden). The genes are cut from suitable plasmids, for instance pPX1604, with appropriate restriction enzymes. Suitable enzymes are EcoR1 and Sal I. The selected genes are purified by cutting from agarose gels. Klenow fragment is used to end repair the purified DNA. The purified gene constructs are cut with suitable restriction enzymes, for instance EcoR1. The cut gene constructs are then ligated into the appropriate restriction sites of selected high expression plasmids. For instance, the cut genes are ligated into the EcoR1 and Sma I restriction enzyme sites of pKK223-3 plasmids (53). The selected plasmids carrying the gene constructs of the present invention are then used to transform suitable microorganisms. For example, E. coli JM105 is transformed with the selected plasmids. Expression of the proteins is detected in a suitable fashion, such as by dot blot analysis using appropriate antisera. For example, the desired transformants were screened for expression of the gene encoding a NH₂-terminal fragment of M24-LT-B carrier (subunit B of E. coli labile toxin) by dot blot analysis using rabbit antisera against a synthetic peptide of M24, SM24 (1-29) C and rabbit antiserum against purified LT-B (16), kindly provided by Dr. John Clements of Tulane University. The appropriate positive transformants barboring the selected plasmids carrying the genes of the present invention are selected for expression and purification of the recombinant protein antigens of the present invention.

The vaccine compositions of the invention include the antigens of the invention and biologically acceptable diluents or adjuvants. The compositions are suitable for eliciting opsonic and/or protective antibodies to serotypes of M protein of Group A streptococcus. The administered compositions of the present invention elicit antibodies, without eliciting cross-reactive antibodies to mammalian heart tissue antigens.

Appropriate biologically acceptable diluents or adjuvants for the present composition may be selected from a wide group of such diluents or adjuvants as readily known to one of skill in the art. A non-limiting example of a diluent is phosphate-buffered saline. The compositions may be administered singly or as a mixture or cocktail.

Another aspect of the present invention are hybrid or fusion genes which have been constructed which encode the antigens of the present invention. The fusion genes code for the antigens of the invention, constituted as described above, of amino acid fragments linked to the selected carrier. The genes are inserted into suitable self-replicating vehicles, like plasmids. The plasmids containing the genes are then used to transform nonvirulent microorganisms. The transformed microorganisms express the hybrid or fusion protein antigens which are capable of eliciting opsonic and/or protective antibodies against serotypes of Group A streptococcus in immunized mammals, without eliciting cross-reactive antibodies to mammalian heart tissue antigens.

One method provides for administration of the compositions to mammals, in particular humans, to elicit opsonic and/or protective antibodies directed to epitopes present in the hybrid antigens of the present invention. No antibodies cross-reactive with heart tissue antigens are elicited. The method comprises administering orally to said mammal, in an amount effective to confer immunity against Group A streptococci infection, a therapeutic composition which comprises a biologically acceptable carrier and a non-virulent, live bacterium as described in United States Patent Number 5,124,153 to Beachey et al., and all references therein incorporated herein by reference. The bacterium is transformed with a plasmid encoding and expressing an antigen of the present invention. The antigen is released from the bacterium, whereby protective antibodies to the antigen of the same serotype as the immunizing antigen are elicited, without eliciting antibodies which are cross-reactive with heart tissue antigens. Immunity against streptococci infection is thereby conferred to the mammal.

There is disclosed administering orally multiple therapeutic compositions. Each composition comprises a hybrid antigen of a serotype of streptococcus. The compositions may be administered individually or as a mixture or cocktail of several compositions. In this fashion, a mammal is immunized against one or more rheumatogenic serotypes of Group A streptococcus. Broad spectrum protective immunity may therefore be established against all rheumatogenic streptococci.

Any biologically acceptable carrier may be used. A biologically acceptable carrier may be PBS, as a non-limiting example. Particularly preferred is a dose of the therapeutic composition suspended in 25 ml of PBS, pH7.2 containing 5 mg/ml kanamycin sulfate and 1 mg/ml each of paraaminobenzoic acid (PABA) and 2, 3-dihydrobenzoic acid (DHB).

It is preferable that the plasmids which encode the M protein genes of the present invention be cloned first and expressed in Escherichia coli. Any other enteric bacilli of the coliform group such as Klebsiella or Enterobacter can be used, but normally E. coli is preferred. Therefore the plasmid carrying the M gene is isolated and purified and then a construct is built to transform the desired non-virulent bacteria, such as the araA-S. typhimurium (SL3261). It is to be noted that this mutant strain exhibits a nutritional marker both for PABA and 2,3-DHB. It is to be noted that another desired specie of S. typhimurium is recA-S. typhimurium, particularly strain Ty21a (17).

It may be desired to obtain the M protein gene from a virulent strain of S. pyogenes. However, it is preferable to obtain the gene from an attenuated, non-virulent strain of S. pyogenes, or to fabricate the nucleotide sequence coding for the desired M protein.

The recombinant DNA cloning vectors are not limited for use in a single specics or strain of Salmonella. To the contrary, the vectors are broadly applicable and can be transformed in host cells of other gram negative bacteria such as of the Enterobacteriaceae genus (such as Shigella and Kiebsiella like (Klebsiella pneumoniae; Enterobacter like Enterobacter aerogenes). Salmonellae, such as Salmonella arizona, and Citrobacter may be used if appropriately rendered non-virulent or attenuated.

Common Salmonella species which may be used when attenuated and rendered non-virulent include the following: S. paratyphi A, S. schottmulleri,S. typhimurium, S. paratyphi C, S. choleraesuis, S. montevideo, S. newport, S. typhi, S. enteritidis, S. gallinarum, and S. anatum.

There may also be used as host for the recombinant DNA cloning vectors of the present invention bacteria of the Streptococcus genus which are non-virulent or which have been made non-virulent or attenuated, including streptococci of the immunological groups A-O but generally other than A. Suitable Streptococci which can be used as bacterial host include S. cremoris, S. faecalis, S. salivarius, S. mitior, S. mitis, S. mutans and S. sanguis. Particularly preferred is S. mutans, which is non-cariogenic.

Additional appropriate microorganisms which may be attenuated and transformed in accordance with the invention are known (31).

Generally any enteric bacterium may serve as the host bacterium. It is preferable that the host bacterium only survive in the subject long enough to elicit the opsonic response, but generally any bacterial strain that has been attenuated so as not to colonize yet still multiply to a limited degree to elicit antibodies to the protein antigen of the present invention can be used. In a preferred embodiment of the invention the Aro⁻ strain of S. typhimurium is used, which requires two metabolites not found in mammalian tissues, PABA and 2,3-DHB. As a result, the inoculated bacteria die after several generations from a lack of these metabolites.

However any mutated microbial agent with a metabolic deficiency for nutritional compounds not found in the tissues of the subject to be immunized, or one so made by genetic manipulations, may be employed.

It is to be noted that the non-virulent are Salmonella typhimunium SL 3261 which has been transformed with a plasmid containing a recombinant hybrid gene encoding a protein antigen expressed the M 5 protein molecule, which expression is confined almost exclusively to the S. typhimurium cytoplasmic compartment. It is unique and unexpected that an immunogenic and protective surface antigen such as the Streptococcal M protein antigen is expressed in the cytoplasm of the non-virulent host bacterium.

Thus it can be seen that the desired nucleotide sequence which codes for and expresses the protein antigen, which is effective to elicit opsonic and/or protective antibodies to streptococcal serotypes, can be cloned into a variety of hosts. In a broader sense therefore, the transformed host in which the nucleotide sequence is found after replication need not be heterologous with respect to the nucleotide sequence, nor does the sequence need to be heterologous with respect to the microorganisms.

In accordance with a specific embodiment of the method of immunization of a warmblooded animal, it has been shown that a) peroral administration of up to 1.65x10⁹ mutant non-virulent Salmonella containing the plasmid pMK207 encoding an antigen of serotype M5 was well tolerated in mice; b) plasmid mPK207 was extremely stable both in vitro and in vivo; c) the mice receiving the highest dose (10⁹) of bacteria harbored the microorganisms in the liver for as long as three weeks without ill effects; d) the mice immunized orally with non-virulent transformed Salmonella expressing the gene developed opsonic serum antibodies as early as three weeks against serotype M5 Streptococci; and e) the immunized mice were completely protected at three weeks against intra-peritoneal challenges of the homologous serotype M5 (but not the heterologous serotype M24) Streptococci.

It is noteworthy that no cross-reactive immunity is observed when the composition of the invention is administered orally. The cytoplasmic expression of the M protein antigen in the non-virulent bacterium is especially advantageous for this oral administration. The antigen is protected within the cytoplasm of the non-virulent bacterium from the acids of the stomach and other damaging agents until the non-virulent cell dies and releases the antigen, ordinarily in the small intestine, which is the preferred location for delivery of the antigens.

The non-virulent bacterium may also be used as a host for recombinant DNA cloning vectors containing nucleotide sequences which code for and express the immunogenic protein antigens of the present invention which are specifically effective to confer immunity against Streptococcal infections and which are not cross-reactive with human tissue antigens, especially those of the heart.

The therapeutic compositions of the present invention may also be administered parenterally. Mammals, in particular humans, immunized parenterally with a sufficient amount of the therapeutic composition of the present invention develop opsonic and/or protective antibodies directed to the epitopes of the hybrid streptococcal M protein antigen. Non-limiting examples of such parenteral routes of administration are intracutaneous and intramuscular.

For intracutaneous injection, 100-300 µg of hybrid antigen emulsified in complete or incomplete Freund's adjuvant was administered in a mammal. A booster injection of about the same dose in saline was administered about one month later. Blood was obtained prior to the first injection and at two-week intervals thereafter for eight weeks.

A topical method of administration is also provided, namely intranasal.

For intranasal administration, a mammal received about 50 µg to about 10 mg of purified antigen in an appropriate diluent for administration.

In accordance with the invention, the therapeutic composition may be administered singly in series or advantageously in a mixture or cocktail of multiple compositions to elicit broad spectrum immunity versus Group A streptococci.

Other advantages of the invention will appear from the non-limiting materials, methods and examples which follow.

### EXAMPLE 1

Purification of LT-B-M24 hybrid protein. The recombinant LT-B-M24 protein was purified from cell extracts of JM105 (harboring pEC L.T-B-M24) grown overnight in one liter of L-broth supplemented with 75 µg/ml ampicillin, 25 µg/ml streptomycin and 1mM isopropylthiogalactoside (IPTG, Bethesda Research Laboratories, Inc., Bethesda, MD). The cells were pelleted at 7,000Xg and resuspended in 50 ml 100 mM carbonate buffer, pH 11, containing 100 µg/ml lysozyme, 1 mM ethylenediaminetetraacetic acid (EDTA, Sigma Chemical Co., St. Louis, MO) and 100 µg/ml phenylmethylsulfonylfluoride (PMSF, Sigma Chemical Co.) and incubated at 37°C for 30 minutes. The cells were centrifuged at 7,000Xg and the supernatant was dialyzed against distilled water and lyophilized. Purification was performed by loading 50 mg of hybrid protein extract onto a preparative polyacrylamide gel electrophoresis unit (Prep Cell, Model 491, Bio Rad., Inc.) using a 37 mm column and a 9 cm 11% polyacrylamide gel. Six ml fractions were collected and assayed for the presence of recombinant protein by Western blot analysis using rabbit antiserum against pep M24 (8). Fractions containing activity were pooled, dialyzed and lyophilized.

### EXAMPLE 2

Immunization of rabbits. Rabbits were immunized intracutaneously with 300 µg LT-B-M24 protein emulsified in complete Freund's adjuvant. A booster injection of the same dose in saline was given four weeks later. Blood was obtained prior to the first injection and at two-week intervals thereafter for eight weeks.

### EXAMPLE 3

Assay for M protein antibodies. Rabbit antisera were assayed for the presence of M protein antibodies by ELISA using LT-B, pep M24 or SM24 (1-29)C as solid phase antigens, as previously described (30, 38). Opsonic antibodies against type 24 streptococci were assayed by in vitro opsonophagocytosis tests (10). Briefly, 0.1 ml of test serum was added to 50 µl of a standard suspension of streptococci. 0.4 ml heparinized, non-immune normal human blood was added and the mixture was rotated end-over-end for 30 minutes. The presence of opsonic antibodies was estimated by counting the percentage of neutrophils with associated streptococci (percent opsonization) on stained smears (10). Indirect bactericidal assays were performed using the same mixture as described above except that fewer streptococci were added (9). The tubes were rotated for three hours and pour plates were made using 0.1 ml of the test mixture in 5% sheep blood agar. CFU of streptococci surviving were counted after incubating overnight at 37°C.

### EXAMPLE 4

Assay for heart-crossreactive antibodies. Rabbit antisera against LT-B-M24 were screened for the presence of heart-crossreactive antibodies by indirect immunofluorescence assays using thin sections (4µ) of human myocardium, as previously described (28).

### EXAMPLE 5

Mouse protection tests. Passive mouse protection tests were performed as previously described (9). Briefly, Balb/c mice were injected intraperitoneally with 0.5 ml test serum, and 24 hrs later, groups of mice were challenged intraperitoneally with 10-fold dilutions of type 24 streptococci. Pour plates were performed to determine the CFU of streptococci that each group received. The LD₅₀ was calculated using the method of Reed and Muench (52).

### EXAMPLE 6

Assay for M protein epitopes that evoke mucosal antibodies broadly protective against infection. Rabbit antiscra were screened for the presence of broadly protective antibodies using passive mouse protection assays (20). Antisera were first tested for their ability to react with the surface M protein of multiple helerologous serotypes of Group A streptococci by ELISA. Those that recognized M protein epitopes in their native conformations were then used to passively protect mice against intranasal challenge infections. Antibodies were absorbed to virulent streptococci and mice were challenged intranasally with 10⁷ CFU. Throat cultures were obtained on alternate days and deaths were counted over the ensuing 14 days.

By way of the Examples, it is shown that the antigens of the present invention elicit opsonic and/or protective antibodies directed to epitopes on the antigens, and confer immunity to immunized mammals against Group A streptococci. The antigens may be advantageously mixed to form a cocktail.

### MATERIALS AND METHODS

Construction and expression of LT-B-M24 fusion gene. Plasmid pPX1604, which contains the gene for LT-B, was kindly provided by Robert Brey, Praxis Biologics, Rochester, NY. pPX1604 is a derivative of pJC217 (16) and was modified to contain a small polylinker with three endonnclease restriction sites at the 3' end followed by transcription terminators in each reading frame (16). The M24 component of the hybrid gene consisted of a pair of oligonucleotides that were synthesized using an automated DNA synthesizer (ABI, model 381A). The oligonucleotides copied the first 36 base pairs of the emm24 gene and included the right hand side of an NcoI site on the 5' end. The oligonucleotides were mixed in equimolar ratios in ligation buffer, heated to 65° and allowed to anneal at ambient temperature. Plasmid pPX1604 was digested with NcoI and EcoRV and the cut plasmid was purified from agarose gels over glassmilk (Geneclean, Bio101, La Jolla, CA). The synthetic M24 oligonucleolide pair was then ligated into the Ncol and EcoRV sites of pPX1604. The ligation mixture was used to transform E. coli strain JM105. Transformants were screened for expression of M24 and LT-B by dot blot analysis using rabbit antisera against a synthetic peptide of M24, SM24 (1-29)C, and rabbit antiserum against purified LT-B (16). The purified LT-B was kindly provided by Dr. John Clcments, Tulane University.

For high level expression of the fusion protein, the LT-B-M24 gene was inserted into pKK223-3 (Pharmacia, Uppsala, Sweden). The hybrid gene was cut from pPX1604 with EcoR1 and Sall and the fragment was purified by cutting from agarose gels. Klenow fragment (53) was used to end repair the purified DNA which was then cut with EcoR1 and ligated into the EcoR1 and Smal restriction enzyme sites of pKK223-3 (53). The ligation mixture was used to transform JM105 and expression of the LT-B-M24 hybrid protein was detected by colony blots as described above. One positive transformant harboring pKK223-3 that contained the hybrid LT-B-M24 gene (pEC.LT-B-M24) was selected for expression and purification of the recombinant protein.

DNA sequencing. The LT-B-M24 gene was sequenced using double-stranded plasmid DNA and ³²P-labeled dNTPs by the dideoxy chain-termination method of Sanger (54). Synthetic oligonucleotides copying the sense strand of p KK223-3 at the EcoR1 site, bases 289-303 of the LT-B gene, and the antisense strands of the HindIII site of pKK223-3 provided sequence data that confirmed the location of the start codon of the LT-B component of the gene, the position of the M24 synthetic oligonucleotide pairs and the NcoI site.

### RESULTS

Immunogenicity of LT-B-M24 hybrid protein. Rabbits immunized with LT-B-M24 developed high titers of antibodies against LT-B, pep M24 and SM24 (1-12)C, as determined by ELISA (Table 1). Interestingly, the antibody titers against the synthetic peptide were equivalent to those against the native pep M24, suggesting that the majority of the M24 antibodies evoked by the LT-B-M24 hybrid recognized M protein epitopes in the native molecule. Immune sera from all three rabbits opsonized type 24 streptococci, indicating that the M protein antibodies were directed against protective epitopes of the surface M protein (Table 1). None of the antisera cross-reacted with human myocardial tissue (data not shown). ELISAs performed at various intervals after the initial injection of LT-B-M24 revealed a brisk antibody response in all three rabbils even after a single intracutaneous dose of LT-B-M24 (Fig. 3).

The rabbit antisera raised against LT-B-M24 contained type-specific, bactericidal antibodies against type 24 streptococci (Table 2). All three antisera had significant bactericidal activity against type 24 streptococci, which in some instances was equivalent to that observed with antiserum against intact pep M24. None of the antisera had bactericidal activity against type 5 streptococci, indicating the type-specificity of the M24 epitopes included in the LT-B-M24 hybrid protein (4). Passive mouse protection tests performed with antisera from rabbit #9146 indicated that antibodies against LT-B-M24 provided significant protection from death compared to pre-immune serum after intraperitoneal challenge with type 24 streptococci (Table 3). In a separate experiment, the LD₅₀ of type 24 streptococci in this assay was 1.5x10⁵ CFU after intraperitoneal injections of preimmune serum, whereas the LD₅₀ after giving LT-B-M24 antiserum was 2.5x10⁶.

It is to be understood that the examples and embodiments described above are not limiting and are for illustrative purposes only and that various modifications or changes in light thereof will be suggested to persons skilled in the art and are to be included within the spirit and purview of this application and the scope of he appended claims.

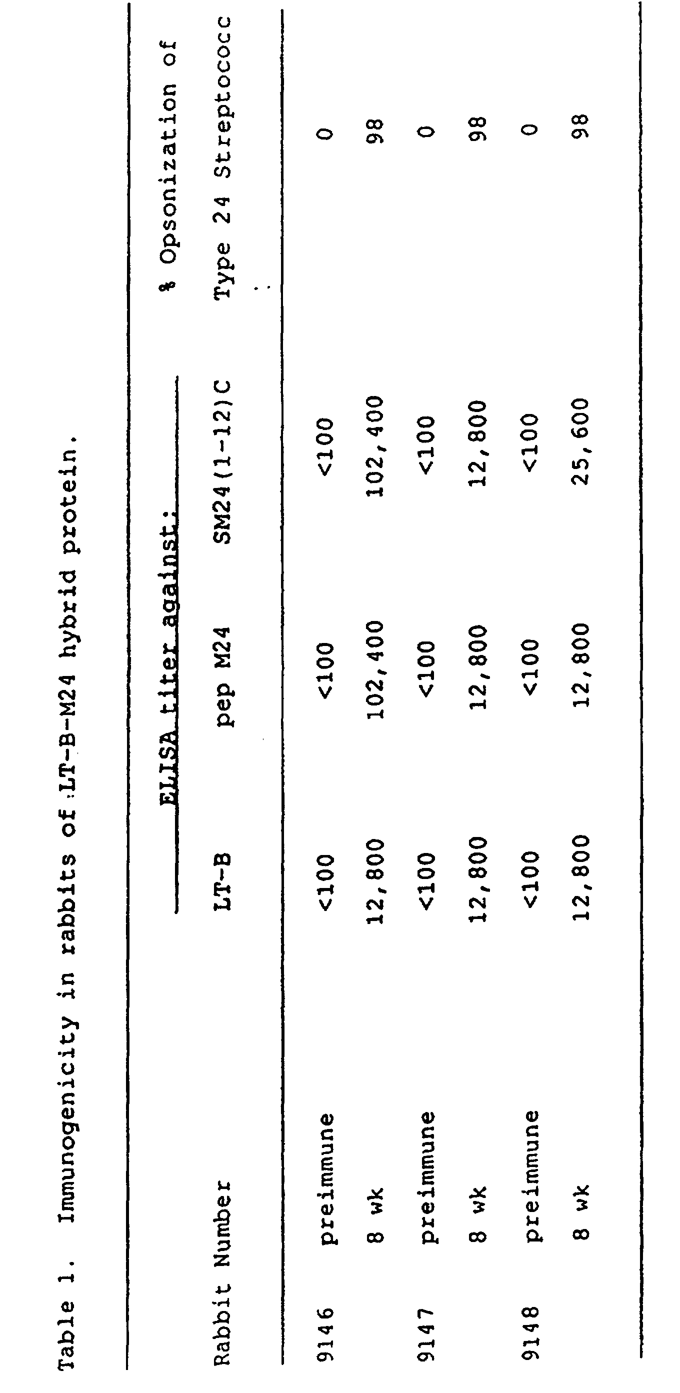

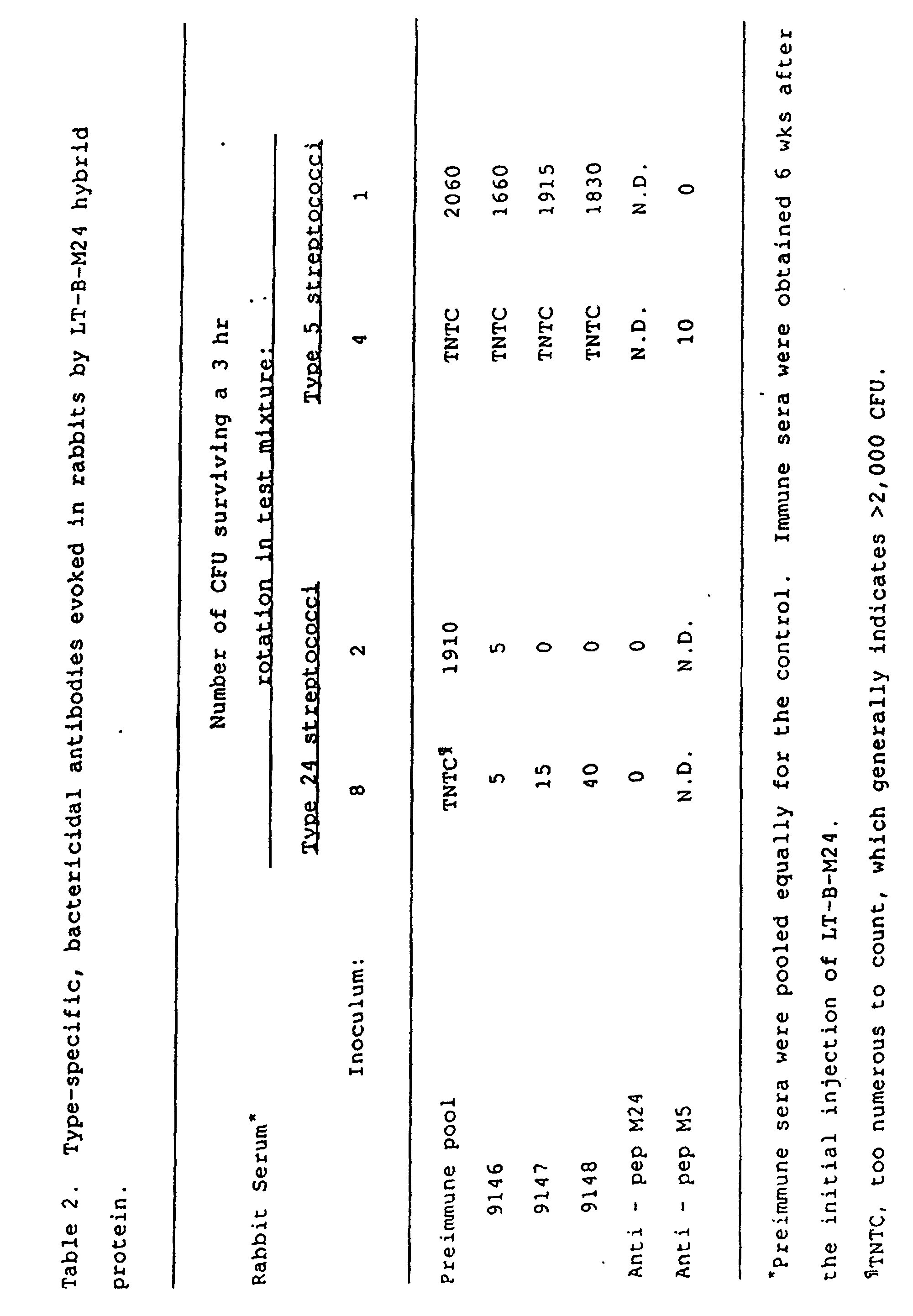

**Table 3.**

| Passive protection of mice challenged intraperitoneally with type 24 streptococci by antiserum against LT-B-M24 hybrid protein. | | |
|---|---|---|
| Antiserum* | #Dead/#Challenged with: | |
| | 13,000 CFU | 100,000 CFU |
| Preimmune | 5/5 | 8/8 |
| Anti - LT-B-M24 | 1/5 (p<.03p)^{¶} | 1/8 (p<.001) |

| | | |
|---|---|---|
| *Mice were given 0.5 ml serum i.p. and 24 hrs later were challenged i.p. with virulent streptococci. Deaths were recorded for one week. | | |
| ^{¶}Statistical analyses were performed using the Fisher exact test on MultiStat Software (Biosoft, Cambridge, UK). | | |

### BIBLIOGRAPHY

1. ATCC Catalogue of Bacterial & Bacleriophages, Editors, Gherna et al., 17th Ed. (1989).
2. ATCC Catalogue of Yeasts, Editors, Jong et al., 18th Ed. (1990).
3. ATCC Catalogue of Recombinant DNA Materials, Edited Maglott et al., 2nd Ed. (1991).
4. Beachey, E. H., et al., "Protective Immunogenicity and T Lymphocyte specificity of a Trivalent Hybrid Peptide Containing NH2-Terminal Sequences of Types 5, 6 and 24 M Proteins Synthesized in Tandem", J. Exp. Med., 166:647 (1987).
5. Beachey, E. H., and Seyer, J. M., "Protective and Nonprotective Epitopes of Chemically Synthesized Peptides of the NH2-Terminal Region of Type 6 Streptococcal M Protein, J. Immunol., 136:2287 (1986).
6. Beachey, E. H., et al., "Repeating Covalent Structure and Protective Immunogenicity of Native and Synthetic Polypeptide Fragments of Type 24 Streptococcal M Protein", J. BioI. Chem., 258:13250 (1983).
7. Beachey, E.H., et al., "Type-Specific, Protective Immunity Evoked by a Synthetic Peptide of Streptococcus Pyogenes M Protein", Nature (London), 292:457 (1981).
8. Beachey, E. H., et al., "Primary Structure of Protective Antigens of Type 24 Streptococcal M Protein", J. BioI. Chem., 255:6284 (1980).
9. Beachey, E. H., et al., "Human Immune Response to Immunization with a Structurally Defined Polypeptide Fragment of Streptococcal M Protein", J. Exp. Med., 150:862 (1979).
10. Beachey, E. H., et al., "Purification and Properties of M Protein Extracted from Group A Streptococci with Pepsin: Covalent Structure of the Amino Terminal Region of the Type 24 M Antigen". J. Exp. Med., 145:1469 (1977).
11. Bellanti, J. A., "Immunology", Saunters Co., (1971).
12. Bisno, A. L., In Reed S.E. and Zabriskie J. B. (eds), "Streptococcal Diseases and the Immune Response, New York, Academic Press 789-803.
13. Bronze, M. S., et al., "Protective and Heart-Crossreactive Epitopes Located within the NH₃-Terminus of Type 19 Streptococcal M Protein", J. Exp. Med., 167:1849 (1988).
14. Bronze, M. S., et al., "Protective and Heart-Crossreactive Epitopes of Type 19 Streptococcal M Protein", Trans. Assoc. Am. Physicians, 100:80-84 (1987).
15. Cavelti. P. A., Proc. Soc. Exp. Med., 60:376 (1945).
16. Clements, J. D., "Construction of a Nontoxic Fusion Peptide for Immunization Against Escherichia coli Strains that Produce Heat-Labile and Heat Stable Enterotoxins", Infect. Immun., 58:1159 (1990).
17. Clements, J. D., "Construction of a Potential Line Aro Vaccine for Typhoid Type Fever and Cholorea-E. coli- Related Diarrheas", Infect. Immun., 46:564-9 (1984).
18. Cunningham, M. W., et al., "A Study of Anti-Group A Streptococcal Monoclonal Antibodies Crossreactivc with Myosin", J. Immunol. 136:293 (1986).
19. Cunningham, M. W., and Russell. S. M., "Study of Heart-Reactive Antibody in Antisera and Hybridoma Culture Fluids Against Group A Streptococci", Infect. Immun., 42:531-538 (1983).
20. Cunningham, M. W., and Beachey E. H., "Peptic Digestion of Streptococcal M Protein I. Effect of Digestion at Suboptimal pH Upon the Biological and Immunological Properties of Purified M Protein Extracts, Infect. Immun., 9:244 (1974).
21. Current Protocols in Molecular Biology, Edited by Ausubel, et al., Greene Associates and Wiley-Interscience (Publishers), Vols. 1 and 2 (1987-88).
22. Current Protocols in Molecular Biology, Edited by Coligan et al., Greene Associates and Wiley-Interscience (Publishers), Vol. 1 (1991).
23. Dale, J. B., and Beachey, E. H., "Localization of Protective Epitopes of the Amino Terminus of Type 5 Streptococcal M Protein", J. Exp. Med., 163:1191 (1986).
24. Dale, J. B., and Beachey, E. H., "Sequence of Myosin-Crossreactive Epitopes of Streptococcal M Protein", J. Exp. Med., 164:1785-1790 (1986).
25. Dale, J. B., and Beachey, E. H., "Multiple Heart-Cross-Reactive Epitopes of Streptococcal M Proteins", J. Exp. Med., 161:113 (1985).
26. Dale, J. B., and Beachey, E. H., "Epitopes of Streptococcal M Proteins Shared with Cardiac Myosin", J. Exp. Med., 162:583-591.
27. Dale, J. B., and Beachey, E. H., J. Exp. Med., 161:122 (1985).
28. Dale, J. B., and Beachey, E. H., "Protective Antigenic Determinant of Streptococcal M Protein Shared with Sarcolemmal Membrane Protein of Human Heart", J. Exp. Med., 156:1165 (1982).
29. Dale, J. B., et al., "Type-Specific Immunogenicity of a Chemically Synthesized Peptide Fragment of Type 5 Streptococcal M Protein", J. Exp. Med., 158:1727 (1983).
30. Dale, J. B., et al., "Heterogeneity of Type-Specific and Cross-Reactive Antigenic Determinants within a Single M Protein of Group A Streptococci", J. Exp. Med., 151:1026 (1980).
31. Davis, et al., Microbiology (Harper & Row, Second edition) (1973).
32. Fischetti, V. A., et al., "Surface Proteins from Gram-Positive Cocci Share Unique Structural Features", New Perspectives on Streptococci and Streptococcal Infections, (G. Orefici, Editor), Gustave and Jena (Publishers) (1992).
33. Fischetti, V. A., "Streptococcal M Protein", Scientific American, 58-65 (1991).
34. Fischetti, V. A., et al., "Streptococcal M6 Protein Expressed in Escherichia coli. Localization, Purification and Comparison with Streptococcal-Derived M Protein", J. Exo. Med., 159:1083 (1984).
35. Goroncy-Bermes, P., et al., "Monoclonal Antibody Against Human Renal Glomeruli Crossreacts with Streptococcal M Protein", Infect. Immun., 55:2416-2419 (1987).
36. Guthrie & Fink, "Guide to Yeast Genetics and Molecular Biology", Method in Enzymology, Academic Press (1991).
37. IBI Catalog, Kodak (1990).
38. Jones, K. F., and Fischetti, V. A., "The Importance of the Location of Antibody Binding on the M6 Protein for Opsonization and Phagocytosis of Group A M6 Streptococci", J. Exp. Med., 167:1114 (1988).
39. Kaplan, M. H., and Meyeserian M., Lancet, 1:706 (1962).
40. Kotb, M., et al., "Stimulation of S-adenosylmethionine Synthetase in Human Lymphocytes by Streptococcal M Protein", J. Immunol. 139:202-206 (1987).
41. Kraus, W., et al., "Autoimmune Sequence of Streptococcal M Protein Shared with the Intermediate Filament Protein, Vimentin", J. Exp. Med., 169:481-492 (1989).
42. Kraus, W., and Beachey, E. H., "Renal Autoimmune Epitope of Group A Streptococci Specified by M Protein Tetrapeptide Ile-Arg-Leu-Arg", Proc. Natl. Acad. Sci. USA, 85:4516 (1988).
43. Kraus, W., et al., "Sequence and Type-Specific Immunogenicity of the Amino-Terminal Region of Type 1 Streptococcal M Protein", J. Immunol., 139-3084 (1987).
44. Krisher, K. and Cunningham, M. W., Science 227-413 (1985).
45. Lancefield, R. C., "Current Knowledge of the Type Specific M Antigens of Group A Streptococci", J. Immunol. 89:307 (1962).
46. Lancefield, R. C., "Persistence of Type-Specific Antibodies in Man Following Infection with Group A Streptococci", J. Exp. Med., 110:271(1959).
47. Miller, L., et al., "Antigenic Variation Among Group A Streptococcal M Proteins: Nucleotide Sequence of the Serotype 5 M Protein Gene and its Relationship with Genes Encoding Types 1, 6 and 24 M Proteins", J. Biol. Chem., 263:5668 (1988).
48. Mouw, A. R., et al., "Molecular Evolution of Streptococcal M Protein: Cloning and Nucleotide Sequence of the Type 24 M Protein Gene and Relation to Other Genes of Streptococcus Pyogenes", J. Bacteriol., 170:676 (1988).
49. Podbielshi, et al., "Application of the Polymerase Chain Reaction to Study the M Protein (-like) Gene Family in Beta - Hemolytic Streptococci", Med. Microbiol Immunol., 180:213 (1991).
50. Poirier, T. P., et al., "Humoral and Mucosal Immunity Against Group A Streptococci Evoked by Attenuated aroA Salmonella Typhimurium Expressing Cloned Streptococcal M Protein", J. Exp. Med., 168:25 (1988).
51. Polly, S. M., et al., "Protective Studies with a Group A Streptococcal M Protein Vaccine. II. Challenge of Volunteers After Local Immunization in the Upper Respiratory Tract", J. Infect. Dis., 131:217 (1975)
52. Reed, L. J., and Muench, H., "A Simple Method of Estimating Fifty Percent Endpoints", Am. J. Hyg., 27:493 (1938).
53. Sambrook, J., et al., "Molecular Cloning: A Laboratory Manual", Cold Spring Harbor Laboratory, Cold Spring Harbor, N.Y. (eds.) (1989).
54. Sanger, F., et al., "DNA Sequencing with Chain-Terminating Inhibitors", Proc. Natl. Acad. Sci., USA, 74:5463 (1977).
55. Sargent. S. J., el al., "Sequence of Protective Epitopes of Streptococcal M Proteins Shared with Cardiac Sarcolemmal Membranes", J. Immunol. 139:1285-1290 (1987).
56. Watson, Molecular Biology of the Gene, 3rd Ed., W.A. Benjamin, Inc.
57. Zabriskie, J. B., et al., Clin. Exp. Immunol. 7:147 (197).
58. United States Patent No. 4,284,537 Beachey, (August 18, 1991).
59. United States Patent No. 4,454,121 Beachey, (June 12, 1984).
60. United States Patent No. 5,124,153 Beachey et al., (June 23, 1992).
61. United States Patent No. 4,521,334 Beachey, (June 4, 1985).
62. United States Patent No. 4,597,967 Beachey, (July 1, 1986).
63. United States Patent No. 4,919,930 Beachey et al., (April 24, 1990).
64. United States Patent No. 4,705,684 Beachey, (November 10, 1987).

## Claims

1. A purified recombinant hybrid streptococcal M protein antigen which comprises a carrier fused to an amino acid fragment, the amino acid fragment having an epitope of a serotype of Group A streptococcal M protein which elicits opsonic antibodies to the same serotype, without eliciting cross-reactive antibodies to mammalian heart tissue antigens, wherein said streptococcal M protein is M1.

2. The recombinant hybrid streptococcal M protein of claim 1, wherein the carrier and the fragment are linked in tandem by a linker comprising amino acids.

3. The recombinant hybrid streptococcal M protein according to claim 1, which elicits mucosal antibodies.

4. The recombinant hybrid streptococcal M protein according to claim 1, wherein the carrier is free of an epitope which elicits antibodies to a serotype of streptococcal M protein.

5. The recombinant hybrid streptococcal M protein according to claim 4, wherein the carrier is the B subunit of *E. coli* labile toxin.

6. The recombinant hybrid streptococcal M protein according to claim 4, wherein the carrier is the C-repeat portion of a streptococcal M protein.

7. The recombinant hybrid streptococcal M protein according to claim 4, wherein the carrier is the COOH-terminal portion of the protein.

8. The recombinant hybrid streptococcal M protein according to claim 2 wherein the linker is Pro-Gly-Asn-Pro-Ala-Val-Pro.

9. The recombinant hybrid streptococcal M protein according to claim 2, wherein the linker is His-Gly.

10. A composition comprising a mixture of purified hybrid streptococcal M proteins according to any one of claims 1 to 9.

11. The composition according to claim 10, wherein said mixture comprises at least one of serotypes M3, M5, M6, M14, M18, M19, M24, M27, and M29.

12. A purified immunogenic recombinant hybrid M protein according to any one of claims 1 to 10, for use in immunizing a mammal against streptococci infection.

13. Use of a purified immunogenic recombinant multivalent hybrid M protein according to any one of claims 1 to 10 in the manufacture of a medicament for immunizing a mammal against streptococci infections.

14. A recombinant DNA molecule, comprising a nucleotide sequence that encodes a hybrid M protein according to any one of claims 1 to 10.

## Patentansprüche

1. Gereinigtes rekombinantes Hybrid Streptokokken M Protein Antigen, das einen an ein Aminosäurefragment fusionierten Träger umfaßt, wobei das Aminosäurefragment ein Epitop eines Serotyps von Gruppe A Streptokokken M Protein aufweist, das opsonisierende Antikörper gegen denselben Serotyp hervorruft, ohne kreuz-reaktive Antikörper gegen Säuger-Herzgewebe Antigene hervorzurufen, wobei das Streptokokken M Protein M1 ist.

2. Rekombinantes Hybrid Streptokokken M Protein nach Anspruch 1, wobei der Träger und das Fragment durch einen Aminosäuren umfassenden Linker in Tandem verbunden sind.

3. Rekombinantes Hybrid Streptokokken M Protein nach Anspruch 1, das mucosale Antikörper hervorruft.

4. Rekombinantes Hybrid Streptokokken M Protein nach Anspruch 1, wobei der Träger frei von einem Epitop ist, das Antikörper gegen einen Serotyp von Streptokokken M Protein hervorruft.

5. Rekombinantes Hybrid Streptokokken M Protein nach Anspruch 4, wobei der Träger die B-Untereinheit von E. *coli* labilem Toxin ist.

6. Rekombinantes Hybrid Streptokokken M Protein nach Anspruch 4, wobei der Träger der C-repeat-Teil eines Streptokokken M Proteins ist.

7. Rekombinantes Hybrid Streptokokken M Protein nach Anspruch 4, wobei der Träger der COOH-terminale Teil des Proteins ist.

8. Rekombinantes Hybrid Streptokokken M Protein nach Anspruch 2, wobei der Linker Pro-Gly-Asn-Pro-Ala-Val-Pro ist.

9. Rekombinantes Hybrid Streptokokken M Protein nach Anspruch 2, wobei der Linker His-Gly ist.

10. Zusammensetzung, umfassend ein Gemisch von gereinigten Hybrid Streptokokken M Proteinen nach einem der Ansprüchen 1 bis 9.

11. Zusammensetzung nach Anspruch 10, wobei das Gemisch mindestens einen der Serotypen M3, M5, M6, M14, M18, M19, M24, M27 und M29 umfaßt.

12. Gereinigtes immunogenes rekombinantes Hybrid M Protein nach einem der Ansprüche 1 bis 10 zur Verwendung bei der Immunisierung eines Säugers gegen Streptokokken-Infektion.

13. Verwendung eines gereinigten immunogenen rekombinanten multivalenten Hybrid M Proteins nach einem der Ansprüche 1 bis 10 zur Herstellung eines Medikaments zur Immunisierung eines Säugers gegen Streptokokken-Infektionen.

14. Rekombinantes DNA Molekül, umfassend eine Nukleotidsequenz, die für ein Hybrid M Protein nach einem der Ansprüche 1 bis 10 kodiert.

## Revendications

1. Antigène recombiné purifié de protéine M streptococcique hybride, qui comprend un vecteur fusionné avec un fragment d'amino-acide, le fragment d'amino-acide ayant un épitope d'un sérotype de protéine M streptococcique du Groupe A qui produit des anticorps opsoniques dirigés contre le même sérotype, sans produire d'anticorps à réaction croisée dirigés contre des antigènes du tissu cardiaque de mammifère, la protéine M streptococcique en question étant la protéine M1.

2. Protéine M streptococcique hybride recombinée suivant la revendication 1, dans laquelle le vecteur et le fragment sont liés en tandem par un lieur comprenant des amino-acides.

3. Protéine M streptococcique hybride recombinée suivant la revendication 1, qui produit des anticorps muqueux.

4. Protéine M streptococcique hybride recombinée suivant la revendication 1, dans laquelle le vecteur est exempt d'un épitope qui produit des anticorps dirigés contre un sérotype de protéine M streptococcique.

5. Protéine M streptococcique hybride recombinée suivant la revendication 4, dans laquelle le véhicule est la sous-unité B de la toxine labile de E. coli.

6. Protéine M streptococcique hybride recombinée suivant la revendication 4, dans laquelle le véhicule est la partie C-répétée d'une protéine M streptococcique.

7. Protéine M streptococcique hybride recombinée suivant la revendication 4, dans laquelle le véhicule est la partie à terminaison COOH de la protéine.

8. Protéine M streptococcique hybride recombinée suivant la revendication 2, dans laquelle le lieur est Pro-Gly-Asn-Pro-Ala-Val-Pro.

9. Protéine M streptococcique hybride recombinée suivant la revendication 2, dans laquelle le lieur est His-Gly.

10. Composition comprenant un mélange de protéines M streptococciques hybrides purifiées suivant l'une quelconque des revendications 1 à 9.

11. Composition suivant la revendication 10, dans laquelle le mélange comprend au moins l'un des sérotypes M3, M5, M6, M14, M18, M19, M24, M27 et M29.

12. Protéine M hybride recombinée immunogénique purifiée suivant l'une quelconque des revendications 1 à 10, destinée à être utilisée dans l'immunisation d'un mammifère contre une infection par des streptocoques.

13. Utilisation d'une protéine M hybride polyvalente recombinée immunogénique purifiée suivant l'une quelconque des revendications 1 à 10 dans la préparation d'un médicament destiné à l'immunisation d'un mammifère contre des infections à streptocoques.

14. Molécule d'ADN recombinée, comprenant une séquence de nucléotides qui code une protéine M hybride suivant l'une quelconque des revendications 1 à 10.
